Europäisches Patentamt

European Patent Office

Office européen des brevets

(19)

(11) Publication number: **0 264 055**
**A2**

(12) # EUROPEAN PATENT APPLICATION

(21) Application number: 87114578.5

(22) Date of filing: 06.10.87

(51) Int. Cl.4: **G01N 33/44**

(30) Priority: 06.10.86 JP 236248/86

(43) Date of publication of application:
20.04.88 Bulletin 88/16

(84) Designated Contracting States:
DE FR GB

(71) Applicant: Murakami, Kenkichi
Komuinshukusha 9-12 68, Kawauchi
Kameoka-Machi
Sendai City Miyagi Prefecture(JP)

(72) Inventor: Murakami, Kenkichi
Komuinshukusha 9-12 68, Kawauchi
Kameoka-machi
Sendai City Miyagi Prefecture(JP)
Inventor: Oikawa, Hidetoshi
2-13-17, Toomizuka
Sendai City Miyagi Prefecture(JP)
Inventor: Suh, Kyung Do
Tohokudai-shukusha 1-13 20-1, Sanjo-machi
Sendai City Miyagi Prefecture(JP)

(74) Representative: Hansen, Bernd, Dr.rer.nat. et
al
Hoffmann, Eitle & Partner Patentanwälte
Arabellastrasse 4
D-8000 München 81(DE)

(54) A method for determining a distribution of polymerization degree between crosslink sites of crosslinked polymers and an apparatus therefor.

(57) In accordance with the present invention, a crosslinked amorphous macromolecule is exposed to air at a temperature range of 100 - 130°C to produce deterioration (oxidation), the chemical stress relaxation is measured, and based on the measured value, the molecular weight distribution (distribution of polymerization degree), i.e., the distribution of polymerization degree between crosslink points of the macromolecule can be calculated.

EP 0 264 055 A2

# A METHOD FOR DETERMINING A DISTRIBUTION OF POLYMERIZATION DEGREE BETWEEN CROSSLINK SITES OF CROSSLINKED POLYMERS AND AN APPARATUS THEREFOR

## Background of the Invention

In order to effectively utilize the right macromolecular substance for the right use, it is necessary to accurately know the properties of the macromolecule. To begin with, it is mandatory to know at least the average molecular weight and the molecular weight distribution (the distribution of polymerization degree) of the macromolecule.

For linear macromolecules, there are already known methods for the determination of average molecule weight. Furthermore, molecular weight distribution can also be determined by methods such as a fractional precipitation and a fractional solution. However, a determination of molecular weight distribution according to said methods takes one or two months for each sample and is extremely boring and prolix. Recently, however, due to the development of gel permeation chromatography (GPC) and high-speed liquid chromatography, it has become possible to quickly determine the molecular weight distribution of macromolecule chains. As a result, their properties can be accurately determined, and the effective utilization of linear macromolecules has been further developed.

However, as for crosslinked macromolecules, it is well known that they are insoluble in all kinds of solvents, and they do not melt when heated. Therefore, it is impossible to apply the conventional molecular weight distribution determining methods which can be used for linear macromolecules.

As a result of research and development by the present inventors, the speedy determination of the molecular weight distribution (distribution of polymerization degree) between the two crosslink points for amorphous crosslinked macromolecules has become possible. Therefore, it is possible to find the properties of crosslinked amorphous macromolecules just as it is for linear macromolecules, and it is believedd that this will greatly contribute to the effective use of these macromolecules.

Among known techniques connected with the molecular weight of crosslinked macromolecules is a determining method for the average molecular weight (average degree of polymerization) between the two crosslink points of crosslinked macromolecules, which corresponds to the average molecular weight of linear macromolecules. This is called the swelling method (see the J. Chem. Phys., 1943 11, page 521). However, there has been no method of determining the molecular weight distribution (distribution of polymerization degree), i.e., the distribution of molecular weight between the two crosslink points of crosslinked macromolecules.

The present invention is one which attampts to provide for the first time a simple and fast method of determining the distribution of molecular weight between crosslink points of crosslinked macromolecules. The basis for this development is theoretical analysis based on chemorheology, a scholarly field in which the present inventors have conducted research for many years.

If raw rubber (a typical example of an amorphous macromolecule) is vulcanized, one obtains vulcanized rubber (a typical example of an amorphous crosslinked macromolecule). If a strain is applied to both ends of a test piece made of raw rubber, the force (f) will gradually decrease due to sliding or diffusion of macromolecular chains within the raw rubber. (This phenomenon is referred to as stress relaxation.) On the other hand, in the case of vulcanized rubber, there is crosslinking between molecular chains, and even if a strain is applied. there is no decrease in (f) as in the case of raw rubber.

However, if a test piece of vulcanized rubber is exposed in air at high temperature, the polymer chains in the vulcanized rubber undergo oxidative degradation (chemical reaction) and breakage or scission takes place (cutting of main molecular chains). As a result, the force (f) remarkably decreases. (This phenomenon is known as chemical stress relaxation.)

Thus, in a vulcanized rubber test piece, the number of molecular chains between the two crosslink points $n(O)$ per unit volume prior to deterioration, the number of molecular chains $n(t)$ after t hours and the number of molecular chain breaks $q_m(t)$ in the test piece after t hours are related in the manner expressed by the following theoretical equation.

$$\frac{n(t)}{n(0)} = e^{-\frac{q_m(t)}{n(0)}} \qquad \cdots\cdots\cdots\cdots (1)$$

However, the polymerization degree between the two crosslink points x in the sample and the rate constant k during a main molecular chain scission reaction are related in the manner expressed by the following experimentally-determined equation.

$$q_m(t) = x \bullet n(O) \bullet k \bullet t \dots \dots \quad (2)$$

Unfortunately, in Equations (1) and (2), the polymerization degree between the two crosslink points is x. and they hold only for certain uniform or uniform network chains. Strictly speaking, the structure of most crosslinked macromolecules is imhomogeneous. Therefore, to be more exact, Equations (1) and (2) become the following Equations (3) and (4).

$$\frac{n_x(t)}{n_x(O)} = e^{\displaystyle -\frac{q_{m \cdot x}(t)}{n_x(0)}} \qquad \dots \dots \dots \dots \quad (3)$$

$$q_{m\,x}(t) = x \cdot n_x(0) \cdot k \cdot t \qquad \dots \dots \dots \dots \quad (4)$$

In Equations (3) and (4), $n_x(O)$ and $n_x(t)$ are respectively the number of molecular chains per unit volume whose polymerization degree between the two crosslink points is exactly x. and the number of molecular chains at t hours after the start of deterioration. $q_{m \bullet x}(t)$ is the number of broken points in the molecular chains.

Based on Equations (3) and (4), the number of remaining unbroken crosslinked molecular chains n(t) after t hours is expressed by the following equation.

$$n(t) = \sum_{x=1}^{\infty} n_x(t) = \sum_{x=1}^{\infty} n_x(o) \cdot e^{\displaystyle \frac{-q_{m \cdot x}(t)}{n_x(0)}} = \sum_{x=1}^{\infty} n_x(o) \cdot e^{-x \cdot k \cdot t} \qquad \dots \dots \dots \quad (5)$$

If both sides of Equation (5) are divided by n(O), one obtains the following equation.

$$\frac{n(t)}{n(o)} = \sum_{x=1}^{\infty} \frac{n_x(o)}{n(o)} \cdot e^{-x \cdot k \cdot t} \qquad \dots \dots \quad (6)$$

However,

$$\frac{n_x(0)}{n(0)}$$

represents the ratio of the number of molecular chains having the polymirization degree x to that of all chains per unit volume. If this is written as M(x), then the preceding equation can be rewritten in the following form.

$$\frac{n(t)}{n(o)} = \sum_{x=1}^{\infty} M(x) \cdot e^{-x \cdot k \cdot t}$$

Generalizing this equation, one gets the following equation.

$$\frac{n(t)}{n(o)} = \int_{o}^{\infty} M(x) \cdot e^{-x \cdot k \cdot t} \cdot dx \qquad \dots \dots \quad (7)$$

3

Where, M(x) corresponds to the distribution of polymerization degree between the two crosslink points.

In research on the chemorheology of vulcanized rubber. the following equations are often used as a basis for the quantitative determination of the deterioration or degradation of crosslinked macromolecules.

Namely, based on the statistical theory of the elasticity of rubber. the stress f(O) acting on deformed rubber whose elongation is maintained at a certain value of extension ratio, is expressed by the following equation.

$$f(O) = n(O)RT(\alpha-\alpha^{-2}) \ldots\ldots \quad (8)$$

Here, R is a gas constant, T is the absolute temperature and $\alpha$ is extension ratio. After t hours, due to deterioration or degradation (chemical change). the crosslinking density has changed from n(O) to n(t), and at this time, the stress is expressed as follows.

$$f(t) = n(t)RT(\alpha-\alpha^{-2}) \ldots\ldots \quad (9)$$

Combining Equations (8) and (9) give the following equation.

$$\frac{f(t)}{f(0)} = \frac{n(t)}{n(0)} \qquad \ldots\ldots\ldots \qquad (10)$$

Combining this equation with Equation (1) gives the following equation.

$$\frac{f(t)}{f(0)} = e^{-\frac{q_m(t)}{n(0)}} \qquad \ldots\ldots\ldots \qquad (11)$$

Thus, it appears that the number of molecular breaks $g_m(t)$ can be easily found from the change in stress f(t)/f(O). However, as a result of numerous experimental investigations, the present inventors discovered that Equation (9) is invalid and accordingly Equations (10) and (11) which are based thereon are incorrect. (See J. Rheo. Soc., Jpn., 9, No.1, 44 - 48 (1987) and J. Polym. Sci., Polym. Lett. Ed., 19, 283 (1981).)

As a result of further research, the following relationship between f(t)/f(O) and n(t)/n(O) was derived.

$$\frac{n(t)}{n(o)} = \left\{ 1 + \frac{\left[\frac{f(o)}{f(t)} - 1\right]}{\left[\frac{B}{n(o)} + 1\right]} \right\}^{-1} \qquad \ldots \quad (12)$$

Here, B is a constant which is inversely proportional to the molecular weight between the two entanglements.

As a result of the above theoretical considerations, it was found that the distribution of polymerization degree (molecular weight) between the two crosslinking points can be determined in the following manner. The variation over time of the deterioration due to stress relaxation, i.e., the relationship between the relative stress f(t)/f(O) and time of a test piece of a crosslinked amorphous macromolecular substance is measured using a simple stress relaxation measuring apparatus, the corresponding value of n(t)/n(O) is found from Equation (12), and the value of distribution of the degree of polymerization i.e., M(x) in Eq(7) is found by using a first or second order approximation equation in Eq(7).

Thus, by measuring the chemical stress relaxation of a crosslinked amorphous macromolecular substance and performing simple calculations, the distribution of polymerization degree between two crosslinks of a crosslinked macromolecule can be easily and rapidly found.

## Summary of the Invention

The present invention comprises two inventions. One is a method of simply and rapidly determining the distribution of polymeriztion degree between the two crosslink points of a crosslinked amorphous macromolecular substance. The second is an apparatus having a special construction for measuring chemical stress relaxation so as to enable the rapid determination of the distribution of polymerization degree between crosslink points.

More particularly the present invention relates to a simple and fast determining method for the distribution of polymerization degree between the two crosslink points of a crosslinked amorphous macromolecule characterized by oxidizing a crosslinked amorphous macromolecule under small strain in air, at high temperature, and finding the distribution of polymerization degree between the two crosslink points by measuring chemical stress relaxation.

If one knows the distribution of polymerization degree between the two crosslink points, then the details of the strength (tensile, bending, frictional, fatigue) of a crosslinked macromolecule become clear. This is because stress concentrations develop centered around the molecular chain having the smallest degree of polymerization between crosslink points, leading to failure.

In conclusion, as a result of the completion of the present invention, light has been shed on the matter of what kind of relationship exists between the properies of a crosslinked macromolecule and its distribution of polymerization degree, on which hithertofore there was no knowledge whatsoever. It is believed that this greatly contributes to the new use and improvement of crosslinked macromolecules.

## Brief Explanation of the Drawings

Figure 1 is a chemical stress relaxation measuring apparatus for use in carrying out the present invention.

Figure 2 shows the construction of an air thermostat used in the measurement of chemical stress relaxation in accordance with the present invention.

Figure 3 is a graph showing the relationship between the degree of polymerization and the distribution of polymerization degree of a test sample obtained as the results of an example of the present invention. The vertical axis is the distribution of polymerization degree between crosslink sites and the horizontal axis is the polymerization degree between crosslinkage.

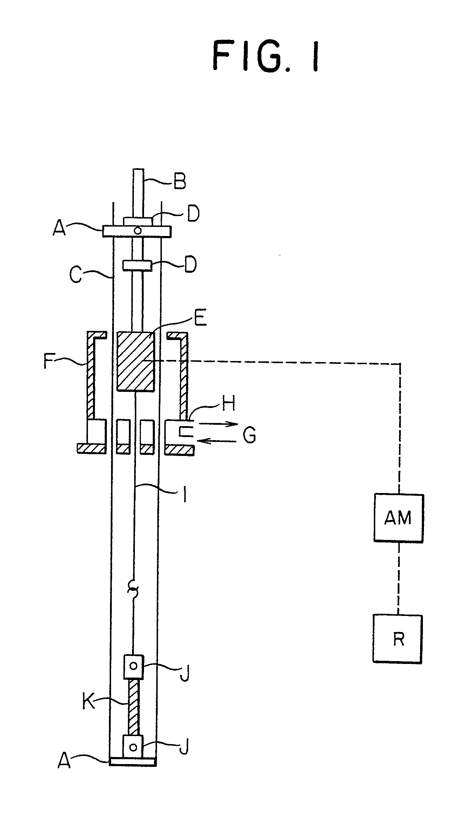

The meanings of the symbols in Figure 1 are as follows.

A: fixed bar
B: stretching slide fixed pillar
C: stretching operating rod
D: stopper
E: strain gauge
F: body
G: cooling water port
H: cooling portion
I: strain gauge inducing wire
J: chuck
K: test piece
AM: dynamic strain meter (or amplifier)
R: recording apparatus

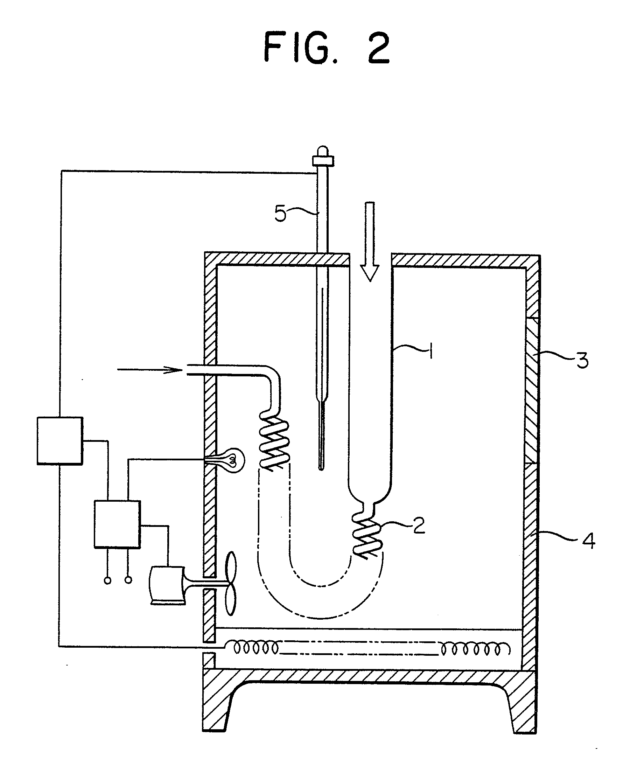

The meanings of the symbols in Figure 2 are as follows.

1: quarts tube for insertion of stress relaxation measuring apparatus
2: air introduction pipe
3: quartz window
4: thermal insulation
5: thermometer (or thermoregulator)

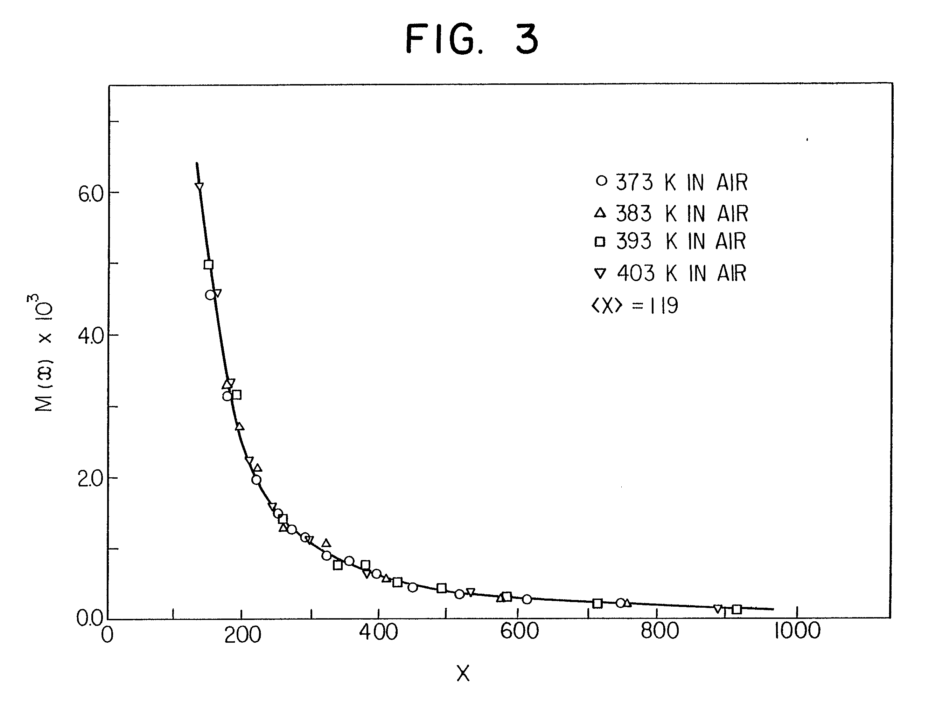

In Figure 3, $M(x) \times 10^3$ on the vertical axis is the distribution of polymerization degree between the two crosslink points, while $x$ along the horizontal axis is the degree of polymerization between the two crosslink points.

5

## Detailed Description of the Invention

A chemical stress relaxation measuring apparatus for carrying out the present invention is a gauge-type stress relaxation measuring apparatus, shown in Figure 1.

The basic structure of this apparatus and the essentials of measurement using it are as follows.

As a test piece, one having a width of 5mm, a length of approximately 3cm, and a thickness of 0.3 - 0.5mm may be used. A test piece (K) is securely installed in an upper and a lower chuck (J). In order to give the test piece a prescribed extension, stoppers (D) are secured at positions corresponding to the upper portion of stretching operating rods (C). By operating the stretching operating rods (C). a prescribed extension is imparted to a test piece. Generally, extension rate of 1.1 - 1.2 is adopted.

The chemical stress relaxation apparatus shown in Figure 1 is inserted into the air thermostat shown in Figure 2, and a test piece is exposed to air heated to a prescribed temperature in the relaxation apparatus.

At the beginning of measurement, in order to obtain temperature equilibrium of the test piece, the relaxation apparatus is inserted into the air thermostat at a prescribed temperature (usually approximately in the range of 80 - 200°C) for about 20 minutes. The measurement is then begun, but measurement of the initial load on the test piece is taken 36 seconds after extension. The elongation of the test piece can be accurately measured through the glass window portion (3) of the air thermostat of Figure 2 using a cathetometer.

The stress relaxation due to deterioration or degradation in air is detected by the strain gauge (E) of Figure 1 and after amplication is recorded by a recorder. The recorder records the stress relaxation with respect to recording time. A computer is connected to the amplifier, and the distribution of polymerization degree between the two crosslink points M(x) can be immediately calculated from the measured stress relaxation data. In this manner, a graph of the distribution of polymerization degree between the two crosslink points of a crosslinked macromolecule against x, the polymerization degree between two crosslinks can be automatically obtained (see Figure 3).

The background of the software used by the computer for calculations and obtaining a graph are as follows.

a. Calculation of the Average Degree of Polymerization Between the Two Crosslink Points (Swelling Method), <x>

After accurate weighting, a small piece of a test material is immersed in benzene at 30°C for 24 - 48 hours. The swollen sample is taken, the benzene adhering to the surface is removed, it is immediately placed into a sample tube and weighed, and the weight of the swollen sample is found.

$$\text{Degree of swelling Q ($\%$)} = \frac{(W-W_0) \times \rho_{rubber}}{\rho_{benzene} \cdot W_0} \times 100$$

$$W_0 = \text{initial weight of the sample}$$
$$W = \text{weight of swollen sample}$$

$\rho$rubber = density of natural rubber (0.915 g/cm³, 30°C)
$\rho$benzene = density of benzen (0.858 g/cm³, 30°C)
Volume fraction Vr = 100/(100 + Q)

It is known that the average degree of polymerization between the two crosslink points by the swelling method is obtained by the following formula. Accordingly, Average polymerization degree between the two crosslink points

$$\langle x \rangle = \frac{\rho_{rubber} \cdot V_o (V_r^{\frac{1}{3}} - \frac{V_r}{2})}{m \left[ \ell_n (1 - V_r) + V_r + \mu V_r^2 \right]}$$

wherein

Vo: molar volume of benzene (89.84 cm³/mol, 30°C)

m: molecular weight of monomer of natural rubber (68 mol⁻¹)

$\mu$: interaction parameter between natural rubber and benzene (0.38)

From the above, the average polymerization degree between crosslinking points of a test material can be found.


b. Calculation of the Reaction Rate Constant k


The curve of chemical stress relaxation is obtained as a chart with measured output voltage as the vertical axis and the chart length as the horizontal axis. If this is converted into a relationship between stress and time on the basis of the previously-corrected full-scale loads and the chart speed, the following relationships are obtained.

Load (F) = (gradations on graph x full-scale load/number of vertical gradations on chart

Stress (f) = (load x g)/cross-sectional area of sample

= (load x g)/(sample width x sample thickness)

Time (t) = chart length · chart speed

Here, g is the gravitational acceleration (980 cm/sec²),

The stress (fo) when the time is zero is taken as a standard and the relative stress f/fo at each time is found. If the initial slope is found in the form $\Delta$ (f/fo)/$\Delta$t, then the reaction rate constant k is expressed as follows.

$$k = \frac{S}{\langle x \rangle} = \frac{\Delta(\,f\,/\,f(o)\,)\,/\,\Delta t}{\langle x \rangle}$$

If the initial slope is difficult to determine, a semilogarithmic plot of f/fo can be made, and k can be directly found from the linear portion. In this case,

$$k = \frac{\Delta(\ell_n\,f\,/\,f(o)\,)}{\Delta t}$$

In this manner, the reaction rate constant k is found, on the horizontal axis this is converted into the natural logarithm ln(kt), and n(t)/n(O) is found using the previously-mentioned Equation (12) and the data for f(t)/f(O). Therefore, if Equation (7) is differentiated by a first or second approximation method based on the relationship between n(t)/n(O) and ln(kt), then a value of the distribution of degree of polymerization between the two crosslink points, M(x) can be obtained. If the above computational steps are incorporated into a program, a microcomputer can directly find M(x).

Next, one example of a so-called embodiment of the present invention will be described in which the distribution of polymerization degree between the two crosslink points of a crosslinked amorphous macromolecule is found using the chemical stress relaxation measuring apparatus of Figure 1.


Example


Natural rubber (pale crepe No. 1) was masticated in an open roll mill at 50± 5°C for 4 minutes, after which 3 weight % of dicumylperoxide was added as a crosslinking agent. Keading was performed for 6 minutes and the mixture was matured for 24 hours, after which it was vulcanized by heating at 145°C under a pressure of 200 kg/cm² for 30 minutes to obtain vulcanized rubber. The vulcanized rubber was then subjected to leaching treatment with hot acetone for 48 hours, after which it was vacuum dried and subjected to experiments.

Test samples were rectangular, measuring approximately 3cm long, 5mm wide, and 0.4mm thick.

These test samples were set in the chemical stress relaxation measuring apparatus shown in Figure 1. and after performing the above-mentioned pretreatment. deterioration in air took place at a temperature in the range of approximately 80 -200°C (absolute temperature of 353 - 473), and a chemical stress relaxation curve was obtained. On the other hand, the average degree of polymerization between crosslinking points (<x> = 119) of the sample was found using the swelling method, and a curve M(x) of the distribution of polymerization degree between the two crosslink points of the sample was found by computer calculations performed on the measured values. The results are shown in Figure 3.

As can be seen from these results, the distribution of polymerization degree between two crosslinkings obtained had no relation to temperature and was consistent.

Effects of the Invention

It is well known that the most inportant properties of a crosslinked macromolecule are the crosslinking density (the average degree of polymerization between the two crosslink points, or the average molecular weight between the two crosslink points) and the distribution of polymerization degree between the two crosslink points. However, hithertofore, only a few methods have been known for finding the average value of the crosslinking density, and there was no method for finding the distribution of polymerization degree between two crosslinkings. This was because the techniques used for linear macromolecules are completely inapplicable to crosslinked macromolecules. However, as a result of the completion of the present invention, by the application of chemorheology which was never even considered in the past, a method has been developed wherein by simply making a crosslinked macromolecule deteriorate under suitable conditions and measuring the chemical stress relaxation, using the measured values, the distribution of polymerization degree between crosslink points is easily, accurately, and rapidly found by microcomputer calculations. As a method of deteriorion or degradation, the technique of thermal oxidation at high temperature was generally used. However, breaking due to deterioration or degradation can take place due to either random scission along a main molecular chain or interchange reaction. Therefore, fundamentally, in accordance with the conditions of the sample, either deterioration by heat or deterioration by light or those by other element can be used. Accordingly, it can be said that the scope of use of the present invention is extremely wide. It can be applied to any amorphous-state crosslinked macromolecule, whether rubber, plastic, or the like.

**Claims**

1. A simple and fast determining method for the distribution of polymerization degree between the two crosslink points of a crosslinked amorphous macromolecule characterized by subjecting a crosslinked amorphous macromolecule to a deteriolating condition under small strain and finding the distribution of polymerization degree between the two crosslink points by measuring chemical stress relaxation.

2. A method according to claim 1 comprising; subjecting crosslinked amorphous macromolecule to deteriolating condition under small strain; measuring chemical stress relaxation; obtaining a reaction rate constant $x$ according to a following equation;

$$k = \frac{\Delta( f / f(o) ) / \Delta t}{\langle x \rangle}$$

(wherein, f(o) represents initial stress and <x> is an average degree of polymerization between two crosslinks) finding a value for

$$\frac{n(t)}{n(0)}$$

according to a following equation;

$$\frac{n(t)}{n(o)} = \left\{ 1 + \frac{\left[ \frac{f(o)}{f(t)} - 1 \right]}{\left[ \frac{B}{n(o)} + 1 \right]} \right\}^{-1}$$

(wherein, n(o) and n(t) represent respectively a number of molecular chains between crosslink points per unit volume at initial stage and that after t hour lapsed, and B represents a constant which is inversely proportional to the molecular weight between two entanglements.)

and determining a distribution of the polymerization degree between two crosslink points by a first or second order approximation of the equation;

$$\frac{n(t)}{n(o)} = \int_{o}^{\infty} M(x) \cdot e^{-x \cdot k \cdot t} \cdot dx$$

(wherein, M(x) represents

$$\frac{n_x(0)}{n(0)}$$

, n(o) is the number of molecular chains between two crosslink points per unit volume and $n_x$ (o) the number of molecular chains whose polymerization degree is X between two crosslink points per unit volume.)

3. A method according to claim 1 or 2 characterized in that said deteriolation condition is oxydation by air in elevated temperature.

4. A method according to claim 1 or 2 characterized in that said deteriolation condition is degradation by photo radiation.

5. A method according to claim 1 to 4 characterized in that crosslinked amorphous macromolecule is rubber vulconizates.

6. A chemical stress relaxation measuring apparatus of the type in which the upper and lower portions of two stretching operating rods which pass through the body of a container having a strain gauge installed therein are each secured to respective fixed bars, a stretching slide fixed pillar which passes through the upper fixed bar is connected to the central, upper portion of the stain gauge, one end of a crosslinked amorphous macromolecular test piece is suspended vertically from its higher portion via a strain gauge stress-inducing wire and a chuck, and the other end of the test piece is secured to the lower fixed bar through a chuck, wherein a dynamic strain meter which reads the gauge and a recording apparatus therefor are appended to said strain gauge, and the test piece is exposed to air at high temperature and a small strain is applied to the test piece by the movement of the stretching operating rods.

# FIG. 1

FIG. 2

FIG. 3

0 264 055